(19) Europäisches Patentamt
European Patent Office
Office européen des brevets

(11) Publication number: **0 316 945 B1**

(12) **EUROPEAN PATENT SPECIFICATION**

(45) Date of publication of patent specification: **08.04.92** (51) Int. Cl.$^5$: **C25B 3/02**

(21) Application number: **88119228.0**

(22) Date of filing: **18.11.88**

(54) **Electrochemical synthesis of 2-methyl-5-pyrazinoic acid.**

(30) Priority: **19.11.87 IT 2269387**

(43) Date of publication of application:
**24.05.89 Bulletin 89/21**

(45) Publication of the grant of the patent:
**08.04.92 Bulletin 92/15**

(84) Designated Contracting States:
**AT BE CH DE ES FR GB LI NL SE**

(56) References cited:
**US-A- 4 496 440**

(73) Proprietor: **MINISTERO DELL' UNIVERSITA' E DELLA RICERCA SCIENTIFICA E TECNOLOGICA**
**76, Lungotevere Thaon di Revel**
**I-00196 Roma(IT)**

(72) Inventor: **Foa', Marco, Dr.**
**19, via del Sabbione**
**I-28100 Novara(IT)**
Inventor: **Forlini, Fabrizio, Dr.**
**67, via Acerbi**
**I-27100 Pavia(IT)**
Inventor: **Gatti, Norberto, Dr.**
**Via Gramsci**
**I-28066 Galliate, Novara(IT)**
Inventor: **Borsotti, Giampiero**
**14, via Pastore**
**I-28100 Novara(IT)**

(74) Representative: **Barz, Peter, Dr. et al**
**Patentanwälte Dr. V. Schmied-Kowarzik**
**Dipl.-Ing. G. Dannenberg Dr. P. Weinhold Dr.**
**D. Gudel Dipl.-Ing. S. Schubert Dr. P. Barz**
**Siegfriedstrasse 8**
**W-8000 München 40(DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services

## Description

The present invention relates to a process for the preparation of 2-methyl-5-pyrazinoic acid by electrochemical oxidation of a functional derivative of 2,5-dimethylpyrazine, on a nickel oxide-hydroxide anode.

2-Methyl-5-pyrazinoic acid is the key-intermediate for the preparation of ACIPIMOX(®) 2-methyl-5-pyrazinoic-1-oxide, an important antihypertensive drug.

From U.K. patent 2,099,820 it is known, how to prepare 2-methyl-5-pyrazinoic acid by consensation of pyruvic aldehyde with diaminodicyanoethylene and subsequent reaction of thus obtained 5-methyl-2,3-dicyanopyrazine with an acid in an aqueous medium.

The reaction between 5-methyl-2,3-dicyanopyrazine with the acid, however, is not selective and gives rise to the formation of equimolecular mixtures of 2-methyl-5-pyrazinoic acid and of 2-methyl-6-pyrazinoic acid,exhibiting the further drawback connected with the separation of said acids.

Moreover from G.B. Barlin "THE PYRAZINES" JOHN WILEY. Ed. page 79 (1982) it is known, how to prepare 2-methyl-5-pyrazinoic acid by oxidation of 2,5-dimethylpyrazine or of 2-methyl-5-oxymethylpyrazine with potassium permanganate.

The above described process, however, proves not to be suitable for an industrial economic production, mostly owing to the considerable amounts of potassium permanganate, which need being used (from 1 to 2 moles of $KMnO_4$ per mole of substrate) and to the problems connected with the separation and getting rid of large amounts of wastes.

Moreover, the reaction between 2,5-dimethylpyrazine with $KMnO_4$ is not selective and gives rise to the formation of considerable amounts of 2,5-dicarboxypyrazine as well.

From J. Kaulen et al. - "Synthesis", 513-516 (1979) it is also known, how to oxidize by electrochemical way primary alcohols on NiO(OH), nickel oxide-hydroxide,anode.

It was now found that 2-methyl -5-pyrazinoic acid can be obtained with high yields and conversions by electrochemical oxidation of 2,5-dimethylpyrazine derivatives on anodes coated with NiO(OH) nickel oxide-hydroxide.

Therefore the object of the present invention is a process for the preparation of 2-methyl -5-pyrazinoic acid consisting in subjecting to electrochemical oxidation a compound having general formula:

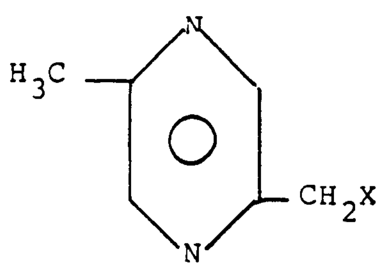

(I)

wherein: X represents OH, Cl, Br,

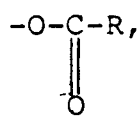

$-O-SO_2-R$,
in which R is a $C_1$-$C_5$ alkyl radical, optionally substituted with one or more atoms of F or Cl, or it is a $C_6$-$C_{12}$ aryl radical, in an electrochemical cell, by using anodes coated with NiO(OH) nickel oxide-hydroxide, in an aqueous alkaline medium, containing at least 5 equivalent moles of a base per mole of compound (I), when X is OH or at least 6 equivalent moles of a base per mole of compound (I), when X is Cl, Br,

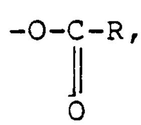

$-O-SO_2-R$

and, optionally an organic solvent for compound (I), which solvent has to be miscible with water and inert under the reaction conditions, at a temperature ranging from 20 to 90 ° C.

The anodic reaction can be schematized as follows:

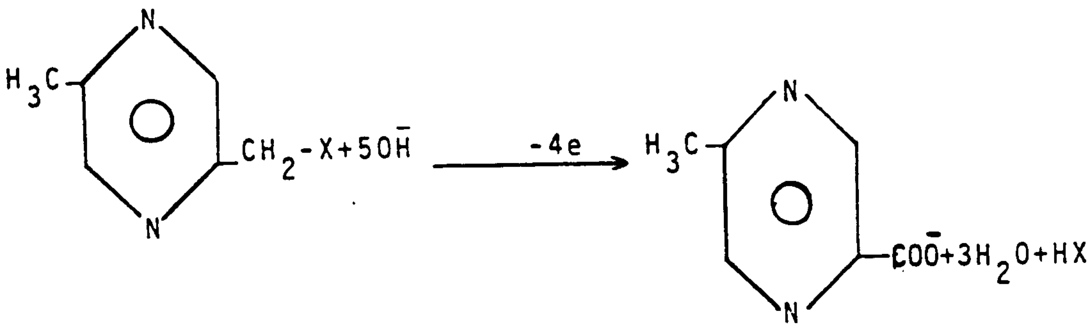


(I) (II)

The concentration of compound (I) in the aqueous basic solution is not critical and can vary between 0.01 and 1 moles/litre, preferably between 0.03 and 0.8 moles/litre.

Hydroxides, carbonates, bicarbonates of alkaline and alkaline-earth metals can be used, for instance, as base.

Tertiary butyl alcohol, tertiary amyl alcohol, acetonitrile can be used, for instance, as organic solvents, which are mixable with water and inert under the reaction conditions.

Such solvents are used preferably to obtain the best yields in 2-methyl-5-pyrazinoic acid (II), when in compound (I),X is Cl or Br.

In the oxidation reaction use can be made of current densities ranging from 5 to 100 m $A/cm^2$, preferably from 8 to 70 m $A/cm^2$.

The quantity of current necessary for the complete conversion of compound (I) into compound (II) can range from 4 to 10 F/mole.

The temperature of oxidation reaction generally ranges from 20 to 90 ° C, preferably from 30 to 70 ° C.

When the reaction of electrochemical oxidation is over, the reaction mixture is acidified up to isoelectric pH of 2-methyl-5-pyrazinoic acid (about pH 1.5).

2-methyl-5-pyrazinoic acid can be extracted, by means of an organic solvent, from the resultant solution, after having salted the solution or after having evaporated the water.

Compounds of formula (I) are known or they can be prepared by known methods, from 2,5-dimethylpyrazine or from its derivatives.

For instance, the compounds of formula (I), when X is Cl or Br, can be prepared by reaction of 2,5-dimethylpyrazine with the customary halogenation agents such as N-chloro (bromo)-succinimide or with sulfuryl chloride.

The compounds of formula (I), when X is

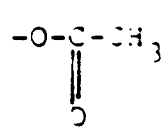

can be obtained by reaction of N-oxide of 2,5-dimethylpyrazine with acetic anhydride, or by exchange reaction between 2-halogenmethyl-5-methylpyrazine with an alkaline acetate.

The compounds of formula (I), when X is -OH, can be obtained by hydrolysis of the corresponding halogen derivative or of the corresponding acyloxymethyl or organic sulfonates.

The compounds of formula (I), when X is $-O-SO_2-R$ can be obtained from the corresponding 2-halogenmethyl-5-methylpyrazines by exchange with an alkaline sulfonate.

The cathodic material of the electrochemical cell is not critical and it generally consists of stainless steel of different kind.

The NiO(OH) nickel oxide-hydroxide anode is prepared, as known from the prior art, by electrolysis, in

an electrochemical cell, of an aqueous alkaline solution of a nickel salt, by changing the electrode polarity by cycles.

The time required for every cycle can range within large limits, from very few seconds to some minutes, taking care that the electrode, which will be used as anode in the reaction of electrochemical oxidation, remains in anodic polarity for a longer time compared with the counter-electrode.

The cycle number may range within large limits as well, according to the desired thickness of NiO(OH) on the electrode and to the modalities, the cyclic polarization of the electrodes is carried out at.

The preferred materials going to make up the electrode, which will be used as work electrode in the oxidation reaction, are stainless steels, nickel, noble metals belonging to the platinum group, in the form of a sheet or a net.

The material for the counter-electrode is not critical and it generally consists of stainless steels of different kind.

The preferred nickel salt is sulfate, other nickel salts may be, however, used, such as, for instance, nitrate.

The salt concentration generally ranges from 0.05 to 2 moles/litre, preferably from 0.08 to 1.5 moles/litre.

The aqueous solution, wherein the nickel salt is dissolved, contains sodium acetate in an amount, that is substantially equimolecular with respect to the nickel salt and hydroxides of alkaline metals ranging from 0.003 to 0.01 moles/litre, preferably from 0.004 to 0.006 moles/litre.

For the preparation of the electrode coated with NiO (OH) use is made of densities of current ranging from 1 to 25 mA/cm$^2$, preferably from 3 to 10 mA/cm$^2$.

A few examples will be given, by way illustration, but not of limitation.

Example 1

Preparation of 2-methyl-5-pyrazinoic acid by oxidation of 2-methyl-5-oxymethylpyrazine.

1) Preparation of the electrode coated with NiO(OH).

99 ml of $H_2O$, 2.93 g (10.3 millimoles) of $NiSO_4.7H_2O$, 0.96 g (11.6 millimoles) of sodium acetate, 1 ml of a solution of 0.5N sodium hydroxide (0.0005 moles) were introduced into an undivided electrochemical cell containing an anode consisting of a nickel net being 24 cm$^2$ in surface and a cathode consisting of a Incoloy 825 wire. The nickel net electrode was polarized anodically for 60 seconds by passing a constant current of 120 m A, afterwards the polarity was inverted for 10 seconds. The cycle was repeated 10 times and at the end the net electrode was kept for 2 minutes under anodic polarity. At the end of this procedure, the nickel net electrode, a NiO(OH) layer had deposited on, was rinsed with water and used in another electrochemical cell for the subsequent oxidation reaction.

2) Electrochemical oxidation.

90 ml of an aqueous solution containing 3.6 g of NaOH and 0.480 g (3.87 millimoles) of 2-methyl-5-oxymethylpyrazine were introduced into an undivided electrochemical cell which contained the anode coated with NiO(OH), prepared as above described and a catode which consisted of an Incoloy 825 wire. The temperature was brought to 40 °C and the electric current was passed at a constant density of 12 mA/cm$^2$. The electrolysis was continued till the passage of 6F per mole of 2-methyl-5-oxymethylpyrazine.

The reaction crude product was acidified with HCl up to a pH of about 1.5, the water was evaporated and the residue was extracted by means of methylethylketone. From the extract, after evaporation of the solvent, 3.6 millimoles of 2-methyl-5-pyrazinoic acid were recovered, with a yield of 93%.

Example 2

100 ml of an aqueous solution containing 6 g of NaOH and 2.56 g of 2-methyl-5-acetoxypyrazine were introduced into an undivided electrochemical cell containing an anode coated with NiO(OH), prepared as described in example 1 and a cathode consisting of an Incoloy 825 wire. The reaction mixture was electrolyzed at 40 °C, with a constant density of current, of 12 mA/cm$^2$ till the passage of 9.000 Coulomb.

When the electrolysis was over, the reaction crude product was acidified with HCl up to a pH of about 1.5, the water was evaporated and the residue was extracted by means of methylethylketone.

From the extract, after evaporation of the solvent, 1.893 g of 2-methyl-5-pyrazinoic acid were recovered, with a yield of 89%.

Example 3

A solution consisting of 15 g of $K_2CO_3$ and 10 g of tertiary butyl alcohol dissolved in 70 ml of water and 0.684 g of 2-chloromethyl-5-methylpyrazine were introduced into an undivided electrochemical cell containing an anode coated with NiO(OH), prepared as described in example 1, and a cathode consisting of an Incoloy 825 wire.

The reaction mixture was electrolyzed at 60 ° C, with a constant density of current of 12 $mA/cm^2$, till the passage of 6F per mole of 2-chloromethyl-5-methylpyrazine.

When the electrolysis was over, the azeotropic mixture $H_2O$/terbutanol was evaporated from the reaction crude product; one diluted slightly with $H_2O$, one acidified with dilute HCl up to a pH of about 1.5, one dried, afterwards the residue was extracted by means of methylethylketone.

From the extract, after evaporation of the solvent, 0.491 g of 2-methyl-5-pyrazinoic acid was recovered, with a yield of 74%.

## Claims

1. A process for the preparation of 2-methyl-5-pyrazinoic acid which comprises subjecting to electrochemical oxidation a compound having genenral formula:

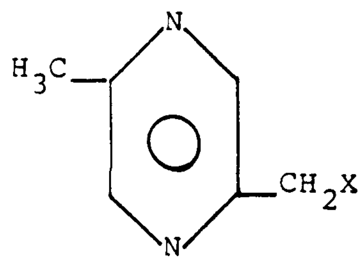

(I)

wherein X is -OH, Cl, Br, -O-CO-R, -O-$SO_2$-R, in which R is a $C_1$-$C_5$ alkyl radical, optionally substituted with one or more atoms of F or Cl, or it is a $C_6$-$C_{12}$ aryl radical, in an electrochemical cell, by using anodes coated with NiO(OH),nickel oxide-hydroxide, in an aqueous medium containing at least 5 equivalent moles of a base per mole of compound (I), when X is OH, or at least 6 equivalent moles of a base per mole of compound (I), when X is Cl, Br, -O-CO-R, -O-$SO_2$-R, at a temperature ranging from 20 to 90 ° C.

2. A process according to claim 1, wherein the aqueous alkaline medium further contains an organic solvent for compound (I), which solvent has to be miscible with water and inert under the reaction conditions.

3. A process according to claim 2, wherein the solvent is selected from tertiary butanol, tertiary amyl alcohol and acetonitrile.

4. A process according to any one of claims 1 to 3, wherein the concentration of compound (I) in the aqueous alkaline solution ranges from 0.01 and 1 mole/litre.

5. A process according to any one of claims 1 to 4, wherein the base is selected from hydroxides, carbonates or bicarbonates of alkaline or alkaline earth metals.

6. A process according to any one of claims 1 to 5, wherein the electrochemical oxidation is carried out at a current density of from 5 to 100 $mA/cm^2$.

## Revendications

1. Un procédé de préparation d'acide 2-méthyl-5-pyrazinoïque qui comprend l'étape consistant à soumettre à une oxydation électrochimique un composé de formule générale:

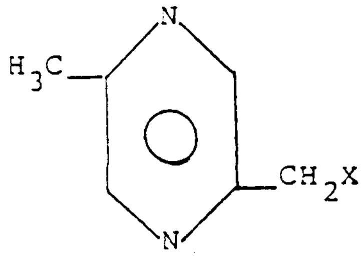

(I)

dans laquelle:

X est -OH, Cl, Br, -O-CO-R, $-O-SO_2-R$, dans lesquelles R est un radical alkyle en $C_1$ à $C_5$, éventuellement substitué avec un ou plusieurs atomes de F ou Cl, ou un radical aryle en $C_6$ à $C_{12}$,

dans une cellule électrochimique, en utilisant des anodes revêtues de NiO(OH), hydroxyde-oxyde de nickel, dans un milieu aqueux contenant au moins 5 équivalents molaires d'une base par mole de composé (I), lorsque X est OH, ou au moins 6 équivalents molaires d'une base par mole de composé (I), lorsque X est Cl, Br, -O-CO-R, $-O-SO_2-R$, à une température de 20 à 90 ° C.

2. Un procédé selon la revendication 1, dans lequel le milieu alcalin aqueux contient de plus un solvant organique pour le composé (I), ledit solvant étant miscible à l'eau et inerte dans les conditions de réaction.

3. Un procédé selon la revendication 2, dans lequel le solvant est choisi parmi le tert-butanol, l'alcool tert-amylique et l'acétonitrile.

4. Un procédé selon l'une quelconque des revendications 1 à 3, dans lequel la concentration de composé (I) dans la solution aqueuse alcaline varie de 0,01 à 1 mole/litre.

5. Un procédé selon l'une quelconque des revendications 1 à 4, dans lequel la base est choisie parmi les hydroxydes, carbonates ou bicarbonates de métaux alcalins ou alcalino-terreux.

6. Un procédé selon l'une quelconque des revendications 1 à 5, dans lequel l'oxydation électrochimique est mise en oeuvre avec une densité de courant de 5 à 100 $mA/cm^2$.

**Patentansprüche**

1. Verfahren zur Herstellung von 2-Methyl-5-pyrazincarbonsäure, welches umfaßt, daß man eine Verbindung der allgemeinen Formel:

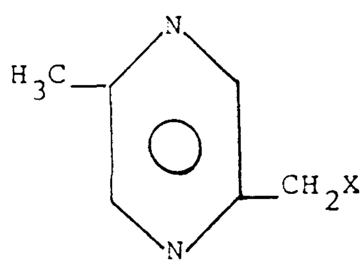

(I)

worin X -OH, Cl, Br, -O-CO-R, oder $-O-SO_2-R$ bedeutet, worin R ein $C_1$-$C_5$ Alkylrest, gegebenenfalls mit ein oder mehreren Atomen F oder Cl substituiert, oder ein $C_6$-$C_{12}$ Arylrest ist,

in einer elektrochemischen Zelle unter Verwendung von Anoden, die mit NiO(OH), Nickeloxid-hydroxid, beschichtet sind, in einen wäßrigen Medium, das mindestens 5 Moläquivalente einer Base pro Mol der Verbindung (I) enthält, wenn X OH ist, oder mindestens 6 Moläquivalente einer Base pro Mol der Verbindung (I) enthält, wenn X Cl, Br, -O-CO-R oder $-O-SO_2-R$ ist, bei einer Temperatur im Bereich von

20 bis 90 ° C einer elektrochemischen Oxidation unterwirft.

2. Verfahren nach Anspruch 1, worin das wäßrige alkalische Medium weiterhin ein organisches Lösungsmittel für die Verbindung (I) enthält, welches Lösungsmittel mit Wasser mischbar und unter den Reaktionsbedingungen inert sein muß.

3. Verfahren nach Anspruch 2, worin das Lösungmittel aus t-Butanol, t-Amylalkohol und Acetonitril ausgewählt ist.

4. Verfahren nach irgendeinem der Ansprüche 1 bis 3, worin die Konzentration der Verbindung (I) in der wäßrigen alkalischen Lösung im Bereich von 0,01 bis 1 Mol/Liter liegt.

5. Verfahren nach irgendeinem der Ansprüche 1 bis 4, worin die Base aus Hydroxiden, Carbonaten oder Bicarbonaten von Alkali- oder Erdalkalimetallen ausgewählt ist.

6. Verfahren nach irgendeinem der Ansprüche 1 bis 5, worin die elektrochemische Oxidation bei einer Ladungsdichte von 5 bis 100 $mA/cm^2$ ausgeführt wird.